**Europäisches Patentamt**

**European Patent Office**

**Office européen des brevets**

(19)

(11) Veröffentlichungsnummer : **0 075 730**
**B1**

# (12) EUROPÄISCHE PATENTSCHRIFT

(45) Veröffentlichungstag der Patentschrift :
**13.06.84**

(21) Anmeldenummer : **82107786.4**

(22) Anmeldetag : **25.08.82**

(51) Int. Cl.³ : **C 07 C 51/56, C 07 C 51/573,
C 07 C 53/12, C 07 C 69/16,
C 07 C 67/29, C 07 C 67/48**

(54) **Verfahren zur Rückgewinnung von Jodverbindungen aus dem Abgas von Carbonylierungsreaktionen.**

(30) Priorität : **23.09.81 DE 3137782**

(43) Veröffentlichungstag der Anmeldung :
**06.04.83 Patentblatt 83/14**

(45) Bekanntmachung des Hinweises auf die Patenterteilung : **13.06.84 Patentblatt 84/24**

(84) Benannte Vertragsstaaten :
**AT BE CH DE FR GB IT LI NL**

(56) Entgegenhaltungen :
**EP-A- 0 013 551
DE-A- 2 940 751
US-A- 1 986 322**

(73) Patentinhaber : **HOECHST AKTIENGESELLSCHAFT
Postfach 80 03 20
D-6230 Frankfurt am Main 80 (DE)**

(72) Erfinder : **Erpenbach, Heinz, Dr.
Oberbuschweg 22
D-5000 Köln 50 (DE)**
Erfinder : **Gehrmann, Klaus, Dr.
Geschwister-Scholl-Strasse 32
D-5042 Erfstadt (DE)**
Erfinder : **Hörstermann, Peter, Dr.
Theodor-Heuss-Strasse 2-4
D-5042 Erfstadt (DE)**
Erfinder : **Kübbeler, Hans-Klaus, Dr.
Bünnagelring 31
D-5357 Swisttal 8 (DE)**
Erfinder : **Kohl, Georg
von Geyr-Ring 61a
D-5030 Hürth (DE)**

**Beschreibung**

Die Erfindung betrifft ein Verfahren zur Rückgewinnung von Jodverbindungen aus dem Abgas, das bei der Umsetzung von Methylacetat und/oder Dimethylether mit Kohlenmonoxid und gegebenenfalls Wasserstoff (Carbonylierung) zu Essigsäureanhydrid und gegebenenfalls Ethylidendiacetat in Gegenwart eines aus Carbonyl-Komplexen von Edelmetallen der Gruppe VIII des Periodensystems, einer Jodverbindung, Essigsäure, einer Organo-Phosphor- oder Organo-Stickstoff-Verbindung sowie gegebenenfalls Verbindungen carbonylbildender Nichtedelmetalle bestehenden Katalysatorsystems, z. B. gemäß DE-A-24 50 965, 28 36 084, 29 39 839 und 29 41 232, anfällt.

Aus der DE-A-30 28 934 ist bereits ein Verfahren zur Gewinnung von Halogenkomponenten aus einem Abgasstrom, der bei der Carbonylierung von Methylacetat und/oder Methylether in Gegenwart eines Metalls aus der Gruppe VIII des Periodensystems der Elemente und einer Halogenverbindung anfällt, bekannt, wobei man einen solchen Abgasstrom in einer Waschzone mit wenigstens einem der bei der Carbonylierungsreaktion anfallenden Produkte, insbesondere Essigsäure, Essigsäureanhydrid und Ethylidendiacetat, in Gegenstromkontakt bringt. Die Rückführung des mit Halogenverbindungen angereicherten Essigsäureanhydrids oder Ethylidendiacetats in die Carbonylierungszone stört jedoch das chemische Gleichgewicht und vermindert die Katalysatorleistung erheblich.

Es ist Aufgabe der vorliegenden Erfindung, eine quantitative Rückgewinnung von Jodverbindungen aus den bei der Carbonylierung von Methylacetat und/oder Dimethylether anfallenden Abgasen ohne die geschilderten Nachteile zu ermöglichen.

Mit den Carbonylierungsprodukten wird besonders bei kontinuierlichen Verfahren ein beträchtlicher Anteil gasförmiger Produkte aus dem Reaktionssystem abgezogen. Nach Trennung von Gas und Flüssigkeit wird der überwiegend aus Kohlenmonoxid bestehende Gasstrom zur Vermeidung einer Anreicherung unerwünschter Nebenprodukte wie Methan und Kohlendioxid sowie von Inertgasen wie Stickstoff in der Gasphase teilweise entfernt. Dieser Abgasstrom enthält Jodverbindungen, die entsprechend ihrem Partialdruck in der Dampfphase vorliegen. Ein Verlust dieser Jodverbindungen durch Verwerfen des abgezogenen Gasstromes ist allein aus wirtschaftlichen Gründen nicht tragbar. Darüberhinaus wirken sich die Jodverbindungen des abgezogenen Gasstromes auf die Beseitigung eines solches Stromes oder die Verwendung der darin enthaltenen Bestandteile, insbesondere des Methans, nachteilig aus. Eine Rückgewinnung der Jodverbindungen aus dem Gasstrom ist deshalb unerläßlich, damit einmal die Jodverbindungen wieder in den Carbonylierungsreaktor zurückgeführt und zum anderen ein gereinigtes Abgas in die Umwelt abgeführt werden kann. In dem Gasstrom liegen die Jodverbindungen überwiegend als Methyljodid neben Ethyljodid und Acetyljodid vor.

Das Verfahren der Erfindung ist nun dadurch gekennzeichnet, daß man den mit den flüssigen Reaktionsprodukten ausgeschleusten Abgasstrom ganz oder teilweise bei Temperaturen von − 40 °C bis + 20 °C im Gegenstrom mit Methylacetat auswäscht.

Weiterhin kann das Verfahren der Erfindung bevorzugt und wahlweise dadurch gekennzeichnet sein, daß

a) man mit einer dem Methylacetatumsatz in der Reaktionszone entsprechenden Menge Methylacetat auswäscht;

b) das Volumenverhältnis von Abgas zu Methylacetat in der Gegenstromwäsche (5-100) : 1, vorzugsweise (10-50) : 1, beträgt;

c) man das zur Wäsche benutzte und mit Jodverbindungen beladene Methylacetat in die Reaktionszone einführt;

d) das Abgas Kohlenmonoxid, Kohlendioxid, Wasserstoff, Stickstoff, Methan, Methylacetat und Jodverbindungen enthält;

d) man den durch Gegenstromwäsche von Jodverbindungen befreiten, überwiegend aus Kohlenmonoxid bestehenden Abgasstrom nach Ausschleusung eines Teilstromes in die Reaktionszone zurückführt.

Das erfindungsgemäße Verfahren erlaubt mit der Verwendung von Methylacetat als Waschflüssigkeit den Einsatz einer an der Carbonylierungsreaktion beteiligten Substanz und vermeidet daher die bei der Verwendung reaktionsfremder Waschflüssigkeiten und deren Einführung in die Carbonylierungszone zu erwartenden Störungen des Reaktionsablaufs.

Durch das Verfahren der Erfindung werden nicht nur die Jodverbindungen quantitativ aus dem Abgasstrom entfernt, sondern auch weitgehend das Methylacetat selbst, das darin in Mengen von 1-2 Vol.% vorhanden ist. Der gereinigte Abgasstrom enthält dann Methylacetat nur noch in Spuren entsprechend dessen Partialdruck und kann infolge seines Gehalts an CO, $H_2$ und $CH_4$ als Brennstoff dienen, soweit er nicht wegen seines hohen CO-Gehaltes in die Carbonylierrungszone zurückgeführt wird.

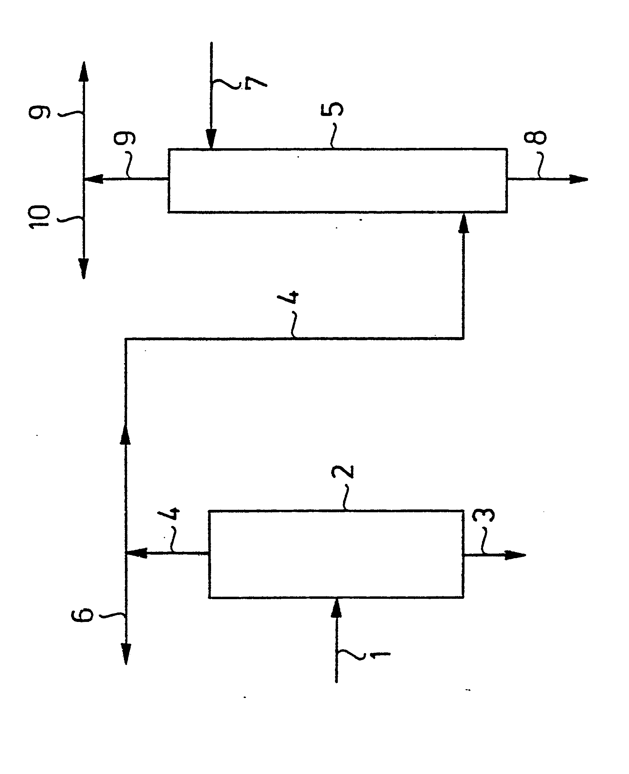

Die Erfindung wird im folgenden anhand der Zeichnung näher erläutert:

Aus dem Carbonylierungsreaktor wird das bei der Carbonylierung von Methylacetat und/oder Dimethylether mit Kohlenmonoxid und ggf. Wasserstoff in Gegenwart eines aus Carbonyl-Komplexen von Edelmetallen der Gruppe VIII des Periodensystems (besonders Rh, Ir, Pd, Ru), Methyljodid, Essigsäure, organischen Phosphor- oder Stickstoffverbindungen sowie gegebenenfalls Verbindungen carbonylbildender Nichtedelmetalle bestehenden Katalysatorsystems erhaltene Reaktionsprodukt abgezogen und über Leitung 1 einer Trennstufe 2 zugeführt. Der aus Gas und Flüssigkeit bestehende Produktstrom hat eine Temperatur von 0-180 °C, vorzugsweise 15-75 °C. Die Trennstufe 2 besteht aus einem Abscheider mit nachgeschalteter Kolonne. Sie wird auf einem Druck von 1-3,5 bar gehalten. Der durch die Leitung 1 zugeführte Produktstrom wird in der Trennstufe 2 in einen flüssigen Anteil, der über Leitung 3 abgezogen wird, und in einen gasförmigen Anteil aufgetrennt. Der gasförmige Anteil enthält dem Partialdruck entsprechend flüchtige Jodverbindungen, die sich durch das erfindungsgemäße Verfahren rückgewinnen lassen. Der am Kopf der Trennstufe 2 vorzugsweise mit einer Temperatur von 15-30 °C ankommende Gasstrom wird über Leitung 4 abgezogen und ganz oder teilweise dem unteren Boden der Waschkolonne 5 zugeführt, die zur Erhöhung des Kontaktes zwischen Gas und Flüssigkeit aus Glockenböden oder einer Füllkörperfüllung besteht. Bei Abzug eines gasförmigen Teilstromes zur Wäsche wird der Hauptstrom über Leitung 6 abgezweigt und zum Carbonylierungsreaktor zurückgeführt. Am Kopf der Waschkolonne 5 wird über Leitung 7 das Methylacetat mit einer Temperatur von + 20 °C bis − 40 °C aufgegeben und das nach oben strömende Abgas im Gegenstrom gewaschen. Das Verhältnis der Volumina Gas : Waschflüssigkeit beträgt (5-100) : 1, vorzugsweise (10-50) : 1. Die mit Jodverbindungen beladene Waschflüssigkeit wird über Leitung 8 abgezogen und zum Carbonylierungsreaktor zurückgeführt. Das jodfreie Abgas verläßt die Waschkolonne über Leitung 9. Bei Beaufschlagung der Wäsche mit dem gesamten Gasstrom wird der überwiegende Teil des gewaschenen Abgases über Leitung 10 zum Carbonylierungsreaktor geführt, während ein Teilstrom, der dem bei teilweiser Beaufschlagung der Wäsche entspricht, über Leitung 9 aus dem System entfernt wird.

In den nachfolgenden Beispielen beziehen sich die Literangaben auf Normalbedingungen (0 °C und 1,013 bar).

Beispiel 1 (Gesamtstromwäsche)

Dem Carbonylierungsreaktor werden 6 250 g/h Reaktionsprodukt entnommen und über Leitung 1 dem unteren Teil der Trennstufe 2 zugeführt. Hier werden bei einer Temperatur von 60 °C die gasförmigen und flüssigen Anteile aufgetrennt. 6 150 g/h flüssige Anteile werden über Leitung 3 abgezogen. Am Kopf der Trennstufe 2 fallen bei einer Temperatur von 20 °C und einem Druck von 1,2 bar 59 l/h Abgas etwa folgender Zusammensetzung in Vol.% an : 76,1 % Co, 3,1 % $CO_2$, 2 % Methan, 1,5 % Wasserstoff, 10,5 % Stickstoff, 5,2 % Methyljodid und 1,6 % Methylacetat. Sie werden über die Leitung 4 dem unteren Teil der aus 20 Glockenböden bestehenden Waschkolonne 5 aufgegeben. Am Kopf der Kolonne 5 werden über Leitung 7 entsprechend dem Methylacetatumsatz in der Carbonylierungszone 1 110 g/h Methylacetat mit einer Temperatur von − 20 °C eingeleitet und das in der Kolonne aufströmende Abgas im Gegenstrom gewaschen. Das Volumenverhältnis von Abgas zu Methylacetat beträgt 49,5 : 1. Über Leitung 8 werden 1 132 g/h Waschflüssigkeit abgezogen, die mit etwa 1,7 Gew.% Methyljodid das gesamte, ursprünglich im Abgas befindliche Methyljodid enthalten. Über Leitung 9 strömen am Kopf der Wäsche 55 l/h methyljodidfreies und im wesentlichen methylacetatfreies Gas ab, wovon 35 l/h über Leitung 1o in den Carbonylierungsreaktor zurück- und 20 l/h über Leitung 9 ausgeschleust werden.

Beispiel 2 (Gesamtstromwäsche)

Dem Carbonylierungsreaktor werden 23 450 g/h Reaktionsprodukt entnommen und über Leitung 1 dem unteren Teil der Trennstufe 2 zugeführt. Hier werden bei einer Temperatur von 70 °C die gasförmigen und flüssigen Anteile aufgetrennt. 23 312 g/h flüssige Anteile werden über Leitung 3 abgezogen. Am Kopf der Trennstufe 2 fallen bei einer Temperatur von 25 °C und einem Druck von 2,4 bar 76 l/h Abgas etwa folgender Zusammensetzung in Vol.% an : 5,6 % Methyljodid, 1,6 % Methylacetat, 73,5 % CO, 1,6 % $CO_2$, 2,6 % Methan, 1,4 % Wasserstoff und 13,6 % Stickstoff. Sie werden über die Leitung 4 der Waschkolonne 5 aufgegeben. Am Kopf der Kolonne 5 werden über Leitung 7 entsprechend dem Methylacetatumsatz in der Carbonylierungszone 2 592 g/h Methylacetat mit einer Temperatur von − 5 °C eingeleitet und das in der Kolonne auf strömende Abgas im Gegenstrom gewaschen. Das Volumenverhältnis von Abgas zu Methylacetat beträgt 27,3 : 1. Über Leitung 8 werden 2 623 g/h Waschflüssigkeit abgezogen, die mit etwa 1 Gew.% Methyljodid das gesamte, ursprünglich im Abgas befindliche Methyljodid enthalten. Über Leitung 9 strömen am Kopf der Wäsche 71 l/h methyljodidfreies Gas ab, wovon 39 l/h über Leitung 10 in den Carbonylierungsreaktor zurück- und 32 l/h über Leitung 9 ausgeschleust werden.

Beispiel 3 (Teilstromwäsche)

Dem Carbonylierungsreaktor werden 4 140 g/h Reaktionsprodukt entnommen und der Trennstufe 2 zugeführt. Hier werden bei einer Temperatur von 50 °C die gasförmigen und flüssigen Bestandteile getrennt. 3 986 g/h flüssige Anteile werden über Leitung 3 entfernt. Am Kopf der Trennstufe 2 fallen bei einer Temperatur von 22 °C und einem Druck von 1,5 bar 128 l/h Abgas etwa folgender Zusammensetzung in Vol.% an : 50,8 % CO, 26,5 % Wasserstoff, 10,2 % Methan, 0,8 % $CO_2$, 4,7 % Stickstoff, 5,5 % Methyljodid und 1,5 % Methylacetat. Davon werden 77 l/h über Leitung 6 direkt in den Reaktor zurückgeführt. Ein Teilstrom von 51 l/h wird über Leitung 4 der Waschkolonne 5 aufgegeben. Am Kopf der Kolonne 5 werden 720 g/h Methylacetat mit einer Temperatur von − 25 °C zugeführt und das in der Kolonne aufströmende Gas im Gegenstrom gewaschen. Das Volumenverhältnis von Abgas zu Methylace-

tat beträgt 66 : 1. Über Leitung 8 werden 740 g/h Waschflüssigkeit abgezogen, die mit 2,35 Gew.% Methyljodid das gesamte, ursprünglich in dem der Wäsche zugeführten Abgasstrom befindliche Methyljodid enthalten. Am Kopf der Wäsche fallen 47,5 l/h methyljodidfreies Abgas an, die über Leitung 9 aus dem System ausgeschleust werden.

**Ansprüche**

1. Verfahren zur Rückgewinnung von Jodverbindungen aus dem Abgas, das bei der Umsetzung von Methylacetat und/oder Dimethylether mit Kohlenmonoxid und gegebenenfalls Wasserstoff zu Essigsäureanhydrid und gegebenenfalls Ethylidendiacetat in Gegenwart eines aus Carbonyl-Komplexen von Edelmetallen der Gruppe VIII des Periodensystems, einer Jodverbindung, Essigsäure, einer Organo-Phosphor- oder Organo-Stickstoffverbindung sowie gegebenenfalls Verbindungen carbonylbildender Nichtedelmetalle bestehenden Katalysatorsystems anfällt, dadurch gekennzeichnet, daß man den mit den flüssigen Reaktionsprodukten ausgeschleusten Abgasstrom ganz oder teilweise bei Temperaturen von − 40 °C bis + 20 °C im Gegenstrom mit Methylacetat auswäscht.

2. Verfahren nach Anspruch 1, dadurch gekennzeichnet, daß man mit einer dem Methylacetatumsatz in der Reaktionszone entsprechenden Menge Methylacetat auswäscht.

3. Verfahren nach Anspruch 1 oder 2, dadurch gekennzeichnet, daß das Volumenverhältnis von Abgas zu Methylacetat in der Gegenstromwäsche (5-100) : 1, vorzugsweise (10-50) : 1, beträgt.

4. Verfahren nach einem der vorhergehenden Ansprüche, dadurch gekennzeichnet, daß man das zur Wäsche benutzte und mit Jodverbindungen beladene Methylacetat in die Reaktionszone einführt.

5. Verfahren nach einem der vorhergehenden Ansprüche, dadurch gekennzeichnet, daß das Abgas Kohlenmonoxid, Kohlendioxid, Wasserstoff, Stickstoff, Methan, Methylacetat und Jodverbindungen enthält.

6. Verfahren nach einem der vorhergehenden Ansprüche, dadurch gekennzeichnet, daß man den durch Gegenstromwäsche von Jodverbindungen befreiten, überwiegend aus Kohlenmonoxid bestehenden Abgasstrom nach Ausschleusung eines Teilstroms in die Reaktionszone zurückführt.

**Claims**

1. Process for recovering iodine compounds from the off-gas originating from the reaction of methyl acetate and/or dimethylether with carbon monoxide and optionally hydrogen to acetic anhydride and optionally ethylidene diacetate in the presence of a catalyst system consisting of carbonyl complexes of noble metals of group VIII of the Periodic System, an iodine compound, acetic acid, an organophosphorus or organonitrogen compound and, optionally, carbonyl-yielding compounds of common metals, wherein the off-gas removed together with the liquid reaction products is wholly or partially subjected to countercurrent scrubbing treatment with methyl acetate at temperatures of − 40 °C up to + 20 °C.

2. Process as claimed in claim 1, wherein the off-gas is scrubbed with a quantity of methyl acetate corresponding to the methyl acetate conversion in the reaction zone.

3. Process as claimed in claim 1 or 2, wherein the off-gas and methyl acetate are used in the countercurrent scrubbing stage in a ratio by volume of (5-100) : 1, preferably (10-50) : 1.

4. Process as claimed in any of the preceding claims, wherein the methyl acetate used for scrubbing and loaded with iodine compounds is introduced into the reaction zone.

5. Process as claimed in any of the preceding claims, wherein the off-gas contains carbon monoxide, carbon dioxide, hydrogen, nitrogen, methane, methyl acetate and iodine compounds.

6. Process as claimed in any of the preceding claims, wherein the off-gas stream freed from iodine compounds by countercurrent scrubbing treatment and consisting predominantly of carbon monoxide is recycled after removal of a portion thereof, into the reaction zone.

**Revendications**

1. Procédé de récupération de composés d'iode du gaz résiduaire que l'on obtient par réaction d'acétate de méthyle et/ou d'éther diméthylique avec le monoxyde de carbone et, éventuellement, l'hydrogène en anhydride acétique et, éventuellement, en diacétate d'éthylidène en présence d'un système catalytique constitué par des complexes carbonyles de métaux nobles appartenant au groupe VIII de la Classification Périodique, un composé d'iode, de l'acide acétique, un composé organophosphoré ou organoazoté et, éventuellement, des composés de métaux non nobles formant des carbonyles, caractérisé en ce que l'on soumet en totalité ou partiellement le gaz résiduaire retiré avec les produits de réaction liquides à un lavage à contre-courant par l'acétate de méthyle à des températures de − 40° à + 20 °C.

2. Procédé selon la revendication 1, caractérisé en ce que l'on effectue le lavage avec une quantité d'acétate de méthyle correspondant à la conversion de l'acétate de méthyle dans la zone de réaction.

3. Procédé selon la revendication 1 ou 2, caractérisé en ce que le rapport en volume gaz résiduaire/acétate de méthyle dans le lavage à contre-courant est de (5-100) : 1, de préférence (10-50) : 1.

4. Procédé selon l'une des revendications précédentes, caractérisé en ce que l'on introduit dans la zone de réaction l'acétate de méthyle utilisé pour le lavage et chargé de composés d'iode.

5. Procédé selon l'une des revendications précédentes, caractérisé en ce que le gaz résiduaire contient du monoxyde de carbone, du dioxyde de carbone, de l'hydrogène, de l'azote, du méthane, de l'acétate de méthyle et des composés d'iode.

6. Procédé selon l'une des revendications précédentes, caractérisé en ce qu'on renvoie dans la zone de réaction, après élimination d'un courant partiel, le courant de gaz résiduaire débarrassé des composés d'iode et consistant essentiellement en monoxyde de carbone.